(19) Europäisches Patentamt
European Patent Office
Office européen des brevets

(11) **EP 0 793 979 B1**

(12) **EUROPÄISCHE PATENTSCHRIFT**

(45) Veröffentlichungstag und Bekanntmachung des Hinweises auf die Patenterteilung:
**18.08.2004 Patentblatt 2004/34**

(51) Int Cl.⁷: **A61N 1/37**, A61N 1/365

(21) Anmeldenummer: **97250059.9**

(22) Anmeldetag: **04.03.1997**

(54) **Anordnung zur Bestimmung der Herzrate**

Apparatus for the determination of the heart rate

Appareil pour la détermination de la fréquence cardiaque

(84) Benannte Vertragsstaaten:
**DE FR GB IT NL SE**

(30) Priorität: **04.03.1996 DE 19609365**

(43) Veröffentlichungstag der Anmeldung:
**10.09.1997 Patentblatt 1997/37**

(73) Patentinhaber: **BIOTRONIK Mess- und Therapiegeräte GmbH & Co Ingenieurbüro Berlin 12359 Berlin (DE)**

(72) Erfinder:
• **Thong, Tran**
**Lake Oswego, Oregon 97035 (US)**
• **Metha, Nawzer**
**Lake Oswego, Oregon 97035 (US)**
• **Wyborny, Paul**
**Lake Oswego, Oregon 97035 (US)**
• **Schaldach, Max, Prof.-Dr.-Ing.**
**91054 Erlangen (DE)**
• **Digby, Dennis**
**Lake Oswego, Oregon 97035 (US)**

(74) Vertreter: **Eisenführ, Speiser & Partner Patentanwälte Rechtsanwälte Spreepalais am Dom Anna-Louisa-Karsch-Strasse 2 10178 Berlin (DE)**

(56) Entgegenhaltungen:
US-A- 4 393 877    US-A- 4 577 633
US-A- 4 895 151    US-A- 4 967 746
US-A- 5 129 393

• PARVIAINEN ET AL: "Ratemeter based on analogue divider", MEDICAL & BIOLOGICAL ENGINEERING AND COMPUTING, , Januar 1978, Band 16, Nr. 1, Seiten 121 - 123

**Beschreibung**

Die Erfindung betrifft eine Anordnung der im Oberbegriff des Anspruchs 1 angegebenen Art.

Die Frequenz der natürlichen Herzaktionen (Herzrate) ist eine Größe, die für die Steuerung von Herzrhythmuskorrekturgeräten - speziell implantierbaren Schrittmachern zur Therapie von Brady- oder Tachyarrhythmien, aber auch von Defibrillatoren bzw. Kardiovertern - von überragender Bedeutung ist. Die korrekte Erfassung dieser Größe, insbesondere der in der Herzkammer erfaßten (ventrikulären) Rate, ist daher spätestens seit der Entwicklung des Bedarfsschrittmachers Gegenstand spezieller Entwicklungsarbeiten.

In deren Rahmen entstanden die bekannten Anordnungen zur automatischen Verstärkungsregelung bzw. zur adaptiven Schwellwertverarbeitung der zur Bestimmung der Herzrate benutzten Herzsignale, mit denen Schwankungen der Herzsignalamplitude (als Haupt-Fehlerquelle bei der Ratenbestimmung) ausgeglichen werden sollen.

Weiterhin hat sich aufgrund der speziellen Signalform der Herzaktionssignale der Einsatz von Refraktärzeitgliedern in Anordnungen zur Bestimmung der Herzrate durchgesetzt.

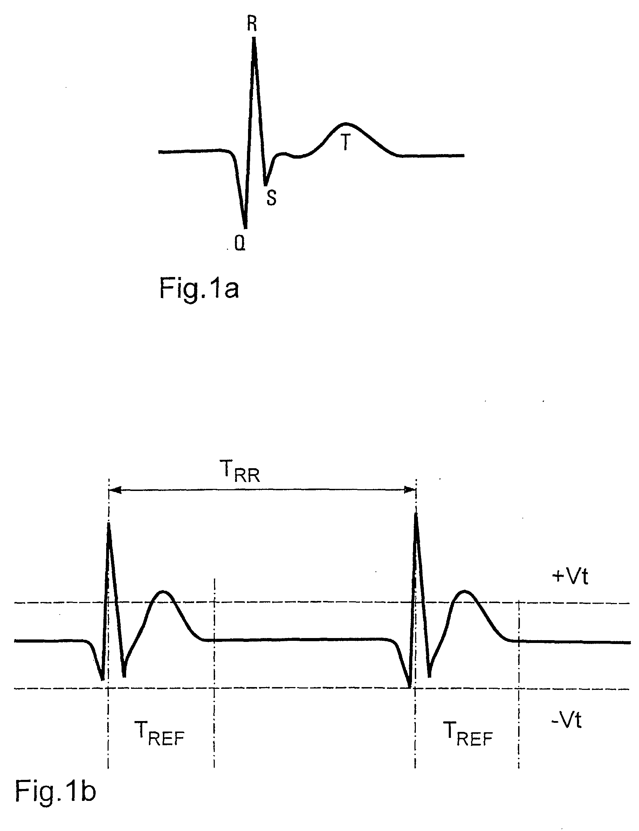

Ein typisches Herzaktionssignal einer Ventrikelaktion, das auch als QRST-Komplex bezeichnet wird, zeigt Fig. 1a. Das Problem, welches den Einsatz eines Refraktärgliedes zweckmäßig macht, ist in Fig. 1b anhand von zwei aufeinanderfolgenden Herzaktionssignalen verdeutlicht: Werden die Signale einer Schwellwertverarbeitung mit einem (beidseits der Signal-Nullinie durch eine gestrichelte Linie mit der Bezeichnung "+Vt" bzw. "-Vt" symbolisierten) Schwellwert unterzogen, d.h. die oberhalb von +Vt (bzw. ggfs. unterhalb von -Vt) liegenden Signalanteile als "Herzschlag" ausgewertet, kann es in praxi speziell zu Fehlmessungen kommen, wenn die maximale Amplitude im T-Abschnitt des QRST-Komplexes oberhalb von +Vt liegt.

Um zu verhindern, daß eine solche "T-Welle" neben der sogenannten "R-Zacke" als separater Herzschlag ausgewertet wird - ein gemeinhin als "Oversensing" bezeichneter Effekt - , ordnet man der Eingangsstufe ein Refraktärglied zu, in dem die in Fig. 1b zwischen den vertikalen strichpunktierten Linien - die die Grenzen der Refraktärzeit bzw. des Refraktärintervalls "REF" angeben - liegenden Abschnitte des Herzaktionssignals ausgeblendet werden. Wie anhand des in der Figur dargestellten Beispiels zu ersehen, wird korrekt der Abstand "RR" der R-Zacken als Herzschlagintervall ausgewertet, und ein "Oversensing" kann somit grundsätzlich vermieden werden.

Aus T. Parviainen et al. "Ratemeter based on analogue divider", Med. & Biol. Eng. & Comput. 1978, 16, 121 ist eine Vorrichtung zur Bestimmung der Herzrate bekannt, bei der optimal eine Ausblendung ("Block") eines Zeitabschnitts von 20 - 70% des Herzschlagintervalls zur Eliminierung von P-Wellen als Störsignal vorgesehen ist.

Es ist auch bekannt, die Refraktärzeit patientenspezifisch zu programmieren und damit der individuellen Herzsignalform weitgehend Rechnung zu tragen.

Speziell im Rahmen bestimmter Tachyarrhythmien und in Übergangsregionen zwischen tachykarden Rhythmusstörungen mit noch deutlich periodischem ("sinusförmigem") Herzsignalverlauf und Herzflimmern (Fibrillationen) können jedoch parallel zueinander starke Amplitudenschwankungen zwischen den Herzsignalkomplexen, aber auch in den relativen Amplituden der einzelnen Signalabschnitte, und Herzratenschwankungen auftreten. Unter solchen Bedingungen kann auch mit einer patientenspezifisch programmierten Refraktärzeit ein "Oversensing" oder - im Gegenteil - die Nichterfassung von R-Zacken ("Undersensing") nicht mehr zuverlässig ausgeschlossen werden

In US 4 393 877 ist ein Herzratendetektor beschrieben, in dem ein Mechanismus zum automatischen Anpassen der Refraktärzeit in Abhängigkeit von der Herzrate vorgesehen werden kann. In welcher Form die Anpassung geschehen soll, d. h. in welcher Weise des Mechanismus aus der Herzrate die Refraktärzeit ermittelt, ist nicht angegeben.

Der Erfindung liegt daher die Aufgabe zugrunde, ein geeignetes Mittel zum Bestimmen der Refraktärzeit zur Verfügung zu stellen.

Diese Aufgabe wird durch eine Anordnung mit den Merkmalen des Anspruchs 1 gelöst.

Die Erfindung schließt den Gedanken ein, anhand eines während des laufenden Betriebes der Anordnung vorab bestimmten Wertes der Herzrate eines vorab erfaßten Herzaktionssignals selbsttätig die aktuell optimale Refraktärzeit für die Herzratenbestimmung einzustellen.

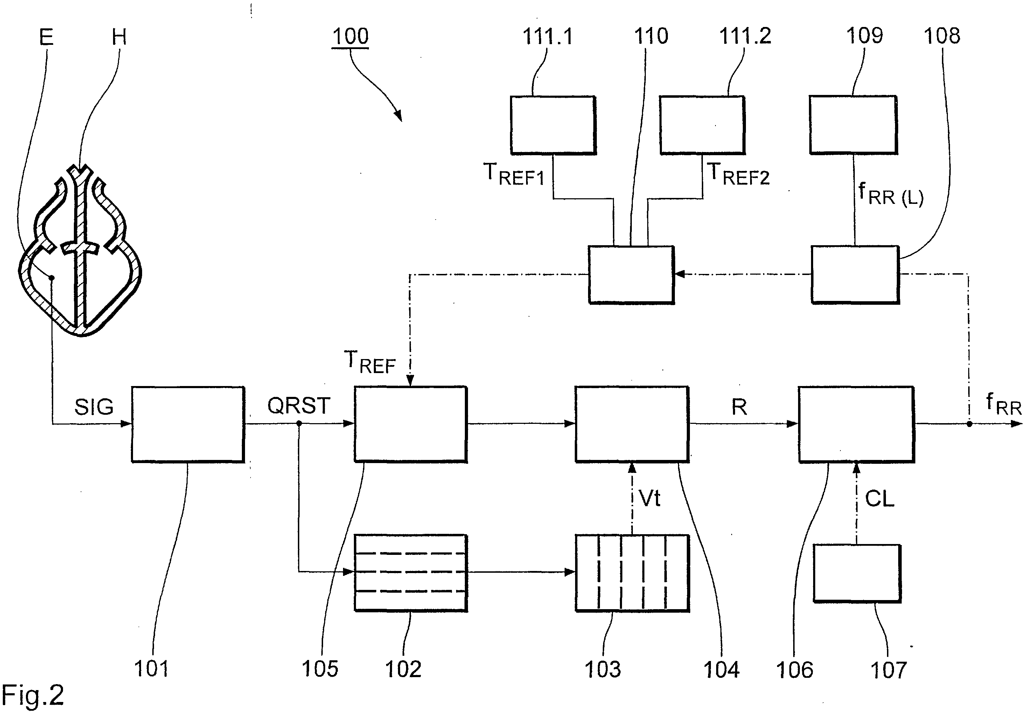

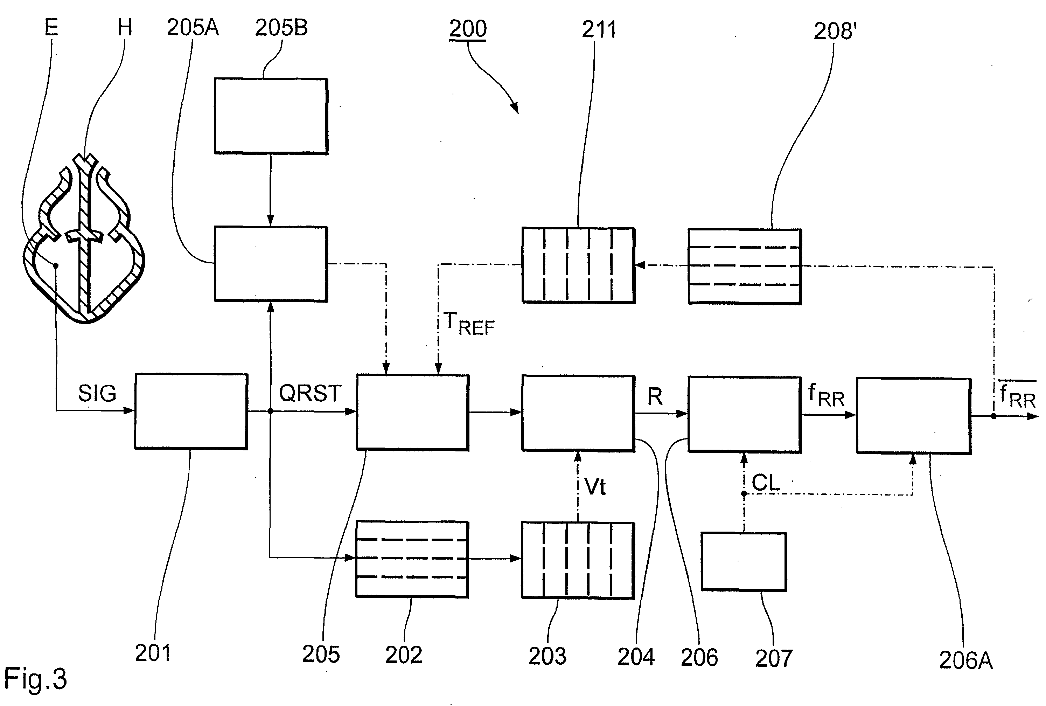

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Die Ausführungsformen, dargestellt in den Figuren 2 und 3, beinhalten nicht alle Merkmale der Erfindung. Es zeigen:

Figur 1a      ein typisches Herzaktionssignal (QRST-Komplex) in vereinfachter Darstellung,

Figur 1b      eine grafische Darstellung zur Erläuterung des Effekts des "Oversensing",

Figur 2      ein stark vereinfachtes Blockschaltbild einer Anordnung nach einer Ausführungsform,

Figur 3      ein stark vereinfachtes Blockschaltbild einer Anordnung gemäß einer weiteren Ausführungsform,

Figur 4     ein stark vereinfachtes Blockschaltbild einer Anordnung gemäß der Erfindung.

Die Figuren 1a und 1b wurden weiter oben erläutert.

Figur 2 zeigt in einem stark vereinfachten Blockschaltbild eine über eine intrakardiale (im Ventrikel angeordnete) Elektrode E mit einem Herzen H verbundene Anordnung 100 zur Herzratenbestimmung in einer Ausführungsform. Die Elektrode E ist mit dem Eingang einer - als solche bekannten - Eingangsstufe 101 mit programmierbarer Filter- und Verstärkungscharakteristik verbunden. Deren Ausgang ist zum einen mit dem Eingang einer Amplitudendiskriminatorstufe 102 verbunden, deren Ausgang mit dem Adreßeingang eines direkt zugreifbaren Schwellwertspeichers 103 verbunden ist. In dessen Speicherplätzen ist jeweils ein Herzsignalamplitudenwert als vorgegebener Schwellwert für die Herzratenbestimmung gespeichert. Der Datenausgang des Schwellwertspeichers 103 ist mit dem Steuereingang eines Schwellwertdiskriminators 104 verbunden. Dessen Dateneingang ist über ein Refraktärglied bzw. eine BlankingSchaltung 105 mit dem Ausgang der Eingangsstufe 101 verbunden, während sein Ausgang mit dem Signaleingang eines Zählers 106 verbunden ist, der durch einen Taktgeber 107 getaktet wird.

Der Ausgang des Zählers 106 ist mit einem Eingang einer Ratenvergleichereinheit 108 verbunden, deren anderer Eingang mit einem Ratengrenzwertspeicher 109 verbunden ist. Der Ausgang der Ratenvergleichereinheit 109 ist mit dem Steuereingang einer Umschalteinheit 110 verbunden, die über zwei Signaleingänge mit den Datenausgängen zweier Refraktärzeit-Festwertspeicher 111.1 und 111.2 und deren Ausgang mit einem Steuereingang des Refraktärgliedes 105 verbunden ist.

Das über die Elektrode E ventrikulär abgegriffene Herzpotentialsignal SIG wird in der Eingangsstufe 101 einer Filterung zur Störbefreiung und einer Verstärkung unterzogen, in deren Ergebnis am Ausgang der Stufe 101 ein Herzaktionssignal QRST bereitsteht, das der Bestimmung der (ventrikulären) Herzrate zugrundegelegt wird. Das Signal QRST wird im Amplitudendiskriminator 102 einer Klassifizierung hinsichtlich der Amplitude der R-Zacke unterworfen und ein digitales Signal ausgegeben, das die Adressierung eines Speicherplatzes des Schwellwertspeichers und die Ausgabe eines vorgespeicherten Schwellwertes Vt an den Schwellwertdiskriminator 104 bewirkt.

Das aufbereitete Signal QRST wird des weiteren im Refraktärglied 105 einer partiellen zeitlichen Ausblendung ("Blanking") für die Dauer einer Refraktärzeit bzw. eines Refraktärintervalls $T_{REF}$, beginnend an der Anstiegsflanke der R-Zacke, unterzogen. Aus dem im Ergebnis der Ausblendung erhaltenen Ausgangssignal der Stufe 105 wird in der sich anschließenden Diskriminatorstufe 104 durch eine als solche wiederum bekannte - Schwellwertverarbeitung ein amplitudennormiertes Impulssignal R erhalten. Die pro Zeiteinheit aufeinanderfolgenden Impulssignale werden im Zähler 106 unter Taktung durch das Taktsignal CL des Taktgebers 107 gezählt, womit sich als Ausgangssignal des Zählers 106 die ventrikuläre Herzrate $f_{RR}$ ergibt.

Das Herzratensignal $f_{RR}$ wird - neben seiner sonstigen Verwendung als Steuersignal eines Herzrhythmuskorrekturgerätes, als Diagnosegröße o.ä. - in der Ratenvergleichereinheit 108 einem Vergleich mit dem im Ratengrenzwertspeicher 109 gespeicherten (bevorzugt patientenspezifisch programmierten) Ratenwert $f_{RR}(L)$ unterzogen. Im Ergebnis des Vergleiches wird ein Schaltsignal an die Umschalteinheit 110 ausgegeben, die im Ansprechen auf das Schaltsignal einen der Festwertspeicher 111.1 oder 111.2 mit dem Refraktärglied 105 verbindet. d.h. diesem einen der vorgespeicherten Refraktärzeitwerte $T_{REF1}$ oder $T_{REF2}$ als gültigen Wert $T_{REF}$ für die Signalausblendung zuführt. Die Bestimmung geeigneter $T_{REF}$-Werte ist weiter unten beschrieben.

Mit der oben beschriebenen Anordnung wird selbsttätig eine zweistufige Anpassung der für die Bestimmung der Herzrate berücksichtigten Refraktär- oder Ausblendzeit in Abhängigkeit von der aktuellen Herzrate vorgenommen und damit die Gefahr eines Over- oder Undersensing verringert.

Figur 3 ist ein stark vereinfachtes Blockschaltbild einer gegenüber Fig. 2 modifizierten Anordnung 200 zur Bestimmung der Herzrate. Funktionsgleiche bzw. -ähnliche Baugruppen wie in Fig. 2 sind mit entsprechenden Bezugsziffern (z.B. 201 entsprechend 101) bezeichnet und werden nachfolgend nicht nochmals erläutert. Die Anordnung unterscheidet sich von der oben beschriebenen zunächst darin, daß anstelle zweier Speicher für je einen Refraktärzeitwert und eines Umschalters ein Direktzugriffspeicher 211 ("look-up table") für eine Mehrzahl von Refraktärzeitwerten vorgesehen ist, der mit dem aus dem aktuellen Ratenzählwert abgeleiteten Ausgangssignal einer mehrstufigen Ratendiskriminatoreinheit 208' adressiert wird. Auf diese Weise wird durch die Auswahl aus einem größeren Vorrat vorbestimmter Werte eine feinere Anpassung der Refraktärzeit an die - in der Stufe 206A zeitlich gemittelte - Herzrate ermöglicht.

Weiterhin ist hier der Eingangsstufe 201 - parallel zum Refraktärglied 205 und zum Amplitudendiskriminator 202 - ein Anstiegsdiskriminator 205A nachgeordnet, der über einen zweiten Eingang mit einem Anstiegs-Schwellwertspeicher 205B verbunden ist und bei Erfassung der Anstiegsflanke einer R-Zacke anhand des den gespeicherten Vergleichs-Schwellwert übersteigenden Anstieges ein Signal zur Aktivierung des Refraktärgliedes 205 ausgibt.

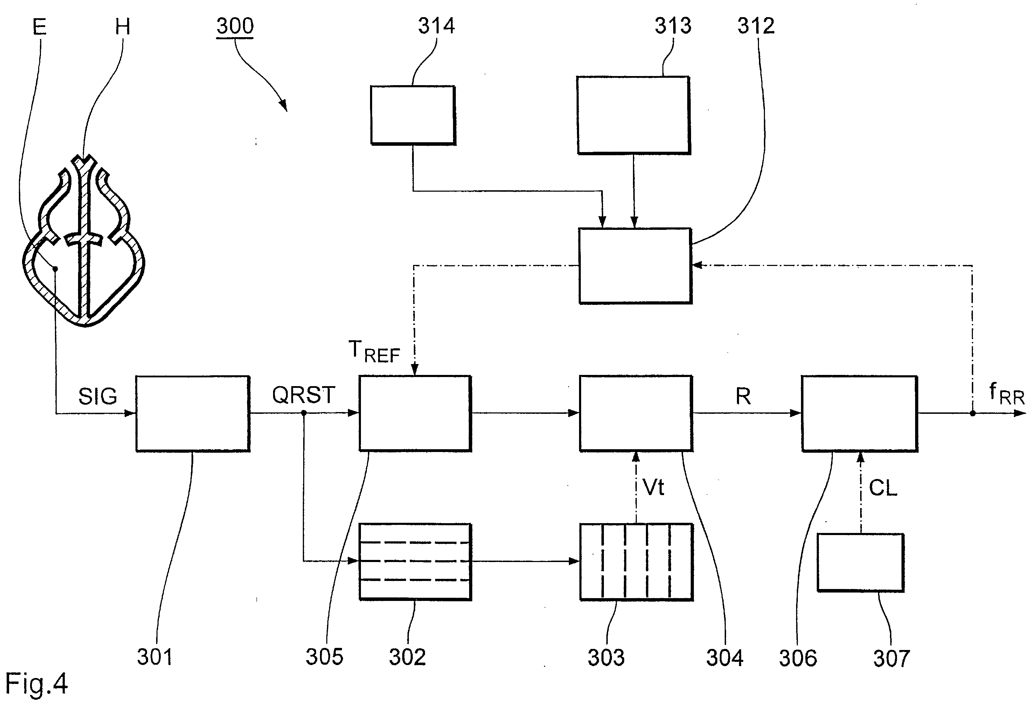

Eine weitere gegenüber Fig. 2 abgewandelte Anordnung 300 gemäß der Erfindung zeigt Fig. 4. Die Anordnung 300 unterscheidet sich von den oben beschriebenen Ratenbestimmungsanordnungen 100 und 200 dadurch, daß anstelle von Speichern für vorprogrammierte Refraktärzeitwerte und diesen zugeordneten Zugriffsmitteln eine direkt vom Ausgangssignal des Zählers 306 gespeiste arithmetische Berechnungseinheit 312 vorgesehen ist, in der aus dem aktuellen Ratenwert jeweils online nach einem in einem Programmspeicher 313 abgelegten Algorithmus und unter Nutzung von in einem Datenspeicher 314 gespeicherten Parameterwerten der optimale Refraktär- bzw. Ausblendzeitwert errechnet wird.

Die Berechnung erfolgt unter Rückgriff auf die in ihrer ursprünglichen Form von H.C. Bazett: "An analysis of the time relations of electrocardiograms", Hearts 7, 353 (1920) angegebene und später von anderen Autoren modifizierte Beziehung

$$QTc = QT + 1{,}75 (f_{RR} - 60) \qquad (1),$$

worin ein Normalwert für $QTC = 410$ Millisekunden bei $f_{RR} = 60$ min-1 sowie etwas geschlechts- und altersabhängig ist.

Ausgehend von (1) kann $T_{REF}$ eingestellt werden zu

$$T_{REF} = QT = QTc - 1{,}75(f_{RR} - 60) \qquad (2),$$

wobei QTc aus elektrophysiologischen Untersuchungen patientenspezifisch bestimmt und gespeichert werden kann. Es kann auch zweckmäßig sein, $T_{REF}$ etwas größer als den sich aus (2) ergebenden Wert zu wählen.

Die vorstehend beschriebenen (oder ähnliche) Anordnungen können als selbständige Geräte oder als Bestandteil von Herzrhythmuskorrekturgeräten, insbesondere Bedarfs- oder Antitachykardie-Schrittmachern, implantierbaren Defibrillatoren (AICD) oder auch automatisch gesteuerten Medikamentendosiergeräten für Antiarrhythmika, realisiert werden.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht.

## Patentansprüche

1. Anordnung zur Bestimmung der Herzrate ($f_{RR}$) (100; 200; 300; 400), insbesondere zur Erkennung einer Tachykardie oder von Fibrillationen, mit einer Elektrode (E) zum Abfühlen von Herzaktionssignalen (SIG), einer mit der Elektrode verbundenen Eingangsstufe (101 bis 104; 201 bis 204; 301 bis 304; 401 bis 404) zur Verarbeitung der Herzaktionssignale, einem Refraktärglied (105; 205; 305; 405) zur Festlegung einer Refraktärzeit ($T_{REF}$) der Anordnung jeweils nach einem vorbestimmten Abschnitt eines Herzaktionssignals und einer mit dem Ausgang der Eingangsstufe bzw. des Refraktärgliedes verbundenen Verarbeitungseinrichtung (106; 206; 306; 406) zur Bestimmung der Rate der unter Ausblendung des während der Refraktärzeit aufgetretenen Anteiles verarbeiteten Herzaktionssignale wobei mindestens mittelbar mit dem Ausgang der Verarbeitungseinrichtung oder der Eingangsstufe verbundene Mittel (108 bis 111.2; 208', 211; 312, 313, 314; 411, 415, 416) zur selbsttätigen Einstellung der Refraktärzeit des Refraktärgliedes in Abhängigkeit von einem vorab bestimmten Wert der Herzrate eines vorab bestimmten Herzaktionssignals vorgesehen sind, **dadurch gekennzeichnet, dass** die Mittel zur selbsttätigen Einstellung der Refraktärzeit (305) eine eingangsseitig mit dem Ausgang der Verarbeitungseinrichtung verbundene arithmetische Berechnungseinheit (312) zur Berechnung der Refraktärzeit nach der vorgegebenen Beziehung

$$T_{REF} = QT = QTc - 1{,}75(f_{RR} - 60)$$

aufweisen, worin QTc =410 Millisekunden einen Normalwert bei $f_{RR} = 60$ Schläge/min und $f_{RR}$ die Herzrate in Schläge/min aufeinanderfolgender R-Zacken darstellt.

2. Anordnung nach einem der vorangehenden Ansprüche, **dadurch gekennzeichnet, dass** die Eingangsstufe eine automatische Verstärkungsregelung oder Mittel (102 bis 104; 202 bis 204; 302 bis 304; 402 bis 404) zur selbsttätigen Einstellung einer Schwellspannung in Abhängigkeit von der Amplitude der Herzaktionssignale umfasst.

3. Anordnung nach einem der vorangehenden Ansprüche, **dadurch gekennzeichnet, dass** die Eingangsstufe zur im wesentlichen vollständigen primären Erfassung des QRST-Signalkomplexes (QRST) von Herzaktionssignalen ausgebildet ist.

4. Anordnung nach einem der vorangehenden Ansprüche, **dadurch gekennzeichnet, dass** die Verarbeitungseinrichtung einen Zähler (106; 206; 306; 406) für erfasste Herzaktionssignale zur Bestimmung der Herzrate aufweist.

5. Anordnung nach einem der vorangehenden Ansprüche, **dadurch gekennzeichnet, dass** das Refraktärglied der Eingangsstufe zugeordnet ist.

6. Anordnung nach einem der vorangehenden Ansprüche, **dadurch gekennzeichnet, dass** eingangsseitig mit der Eingangsstufe verbundene Schaltmittel (205A) zur Aktivierung des Refraktärgliedes jeweils bei Erfassung der Anstiegsflanke der R-Zacke eines Herzaktionssignales vorgesehen sind.

7. Anordnung nach einem der vorangehenden Ansprüche, **dadurch gekennzeichnet, dass** eine mit dem Ausgang des Zählers (206) verbundene Mittelungseinrichtung (206A) zur Bestimmung einer mittleren Herzrate ($f_{RR}$) vorgesehen ist und die Mittel zur selbsttätigen Einstellung der Refraktärzeit (208', 211) mit dem Ausgang der Mittelungseinrichtung verbunden sind.

8. Implantierbarer Herzschrittmacher, insbesondere zur Behandlung von Tachyarrhythmien, mit einer Anordnung nach einem der vorangehenden Ansprüche.

9. Implantierbarer Defibrillator mit einer Anordnung nach einem der vorangehenden Ansprüche.

## Claims

1. An apparatus for determining the heart rate ($f_{RR}$) (100; 200; 300; 400), in particular for detecting tachycardia or fibrillations, comprising
an electrode (E) for sensing heart action signals (SIG),
an input stage (101 to 104; 201 to 204; 301 to 304; 401 to 404) connected to the electrode for processing the heart action signals,
a refractory element (105; 205; 305; 405) for ascertaining a refractory period ($T_{REF}$) of the apparatus in each case after a predetermined segment of a heart action signal and of a processing device (106; 206; 306; 406) connected to the output of the input stage or of the refractory element to determine the rate of the heart action signals processed by blanking out the component that occurred during the refractory period,
wherein means (108 to 111.2; 208', 211; 312, 313, 314; 411, 415, 416) connected at least indirectly to the output of the processing device or the input stage are provided for the automatic adjustment of the refractory period of the refractory element as a function of a previously determined value of the heart rate of a previously determined heart action signal,
**characterised in that** the means for the automatic adjustment of the refractory period (305) comprise an arithmetic calculation unit (312) connected at the input side to the output of the processing device for the calculation of the refractory period in accordance with the predetermined equation

$$T_{REF} = QT = QTc - 1.75(f_{RR} - 60)$$

in which QTc = 410 milliseconds represents a normal value at $f_{RR}$ = 60 beats per minute and $f_{RR}$ represents the heart rate in beats per minute of successive R spikes.

2. An apparatus according to one of the preceding Claims, **characterised in that** the input stage comprises an automatic gain closed-loop control or means (102 to 104; 202 to 204; 302 to 304; 402 to 404) for the automatic adjustment of a threshold voltage as a function of the amplitude of the heart action signals.

3. An apparatus according to one of the preceding Claims, **characterised in that** the input stage is constructed for the substantially complete primary recording of the QRST signal complex (QRST) of heart action signals.

4. An apparatus according to one of the preceding Claims, **characterised in that** the processing device comprises a counter (106; 206; 306; 406) for recorded heart action signals for determining the heart rate.

5. An apparatus according to one of the preceding Claims, **characterised in that** the refractory element is associated with the input stage.

6. An apparatus according to one of the preceding Claims, **characterised in that** switching means (205A) connected on the input side to the input stage are provided to activate the refractory element each time the rising edge of the R spike of a heart action signal is recorded.

7. An apparatus according to one of the preceding Claims, **characterised in that** an averaging device (206A) connected to the output of the counter (206) is provided to determine a mean heart rate ($f_{RR}$) and the means for the automatic adjustment of the refractory period (208', 211) are connected to the output of the averaging device.

8. An implantable cardiac pacemaker, in particular for the treatment of tachycardyarrhymias, having an apparatus according to one of the preceding Claims.

9. An implantable defibrillator having an apparatus according to one of the preceding Claims.

## Revendications

1. Dispositif pour déterminer le rythme cardiaque ($f_{RR}$) (100; 200; 300; 400) notamment pour l'identification d'une tachycardie ou de fibrillations, comportant une électrode (E) servant à détecter des signaux d'action cardiaque (SIG), un étage d'entrée (101 à 104; 201 à 204; 301 à 304; 401 à 404) relié à l'électrode et servant à traiter les signaux d'action cardiaque, un circuit de détermination de la période réfractaire (105; 205; 305; 405) pour déterminer une période réfractaire ($T_{REF}$) du dispositif respectivement selon une section prédéterminée d'un signal d'action cardiaque, et un dispositif de traitement (106; 206; 306; 406), relié à la sortie de l'étage d'entrée ou du circuit de détermination de la période réfractaire, pour déterminer le rythme des signaux d'action cardiaque traités moyennant l'occultation de la composante apparue pendant la période réfractaire, et dans lequel il est prévu des moyens (108 à 111.2; 208', 211; 312, 313, 314; 411, 415, 416), qui sont reliés au moins indirectement à la sortie du dispositif de traitement ou à l'étage d'entrée et sont utilisés pour le réglage automatique de la période réfractaire du circuit de détermination de la période réfractaire, en fonction d'une valeur préalablement déterminée du rythme cardiaque d'un signal d'action cardiaque déterminé au préalable, **caractérisé en ce que** les moyens de réglage automatique de la période réfractaire (305) possèdent une unité de calcul arithmétique (312), qui est reliée côté entrée à la sortie du dispositif de traitement et sert à calculer la période réfractaire conformément à la relation prédéterminée

$$T_{REF} = QT = QTc - 1,75(f_{RR} - 60)$$

QTc = 410 millisecondes représentant une valeur normale pour $f_{RR}$ = 60 battements/mn et $f_{RR}$ étant le rythme cardiaque en battements/mn de pointes R successives.

2.  Dispositif selon l'une des revendications précédentes, **caractérisé en ce que** l'étage d'entrée comprend une unité automatique de régulation d'amplification ou des moyens automatiques (102 à 104; 202 à 204; 302 à 304; 402 à 404) pour régler automatiquement une tension de seuil en fonction de l'amplitude des signaux d'action cardiaque.

3.  Dispositif selon l'une des revendications précédentes, **caractérisé en ce que** l'étage d'entrée est agencé pour réaliser la détection primaire essentiellement complète du complexe QRST (QRST) de signaux d'action cardiaque.

4.  Dispositif selon l'une des revendications précédentes, **caractérisé en ce que** le dispositif de traitement comporte un compteur (106; 206; 306; 406) pour des signaux d'action cardiaque détectés pour la détermination du rythme cardiaque.

5.  Dispositif selon l'une des revendications précédentes, **caractérisé en ce que** le circuit de détermination de la période réfractaire est associé à l'étage d'entrée.

6.  Dispositif selon l'une des revendications précédentes, **caractérisé en ce que** des moyens de commutation (205A) reliés côté entrée à l'étage d'entrée et servant à activer le circuit de détermination de la période réfractaire sont prévus respectivement lors de la détection du flanc montant de la pointe R d'un signal d'action cardiaque.

7.  Dispositif selon l'une des revendications précédentes, **caractérisé en ce qu'**un dispositif de formation de la moyenne (206A), qui est relié à la sortie du compteur (206) est prévu pour déterminer un rythme cardiaque moyen ($f_{RR}$), et les moyens pour le réglage automatique de la période réfractaire (208', 211) sont reliés à la sortie du dispositif de formation de la moyenne.

8.  Stimulateur cardiaque implantable, notamment pour le traitement de tachyarythmie, comportant un dispositif selon l'une des revendications précédentes.

9.  Défibrillateur implantable comportant un dispositif selon l'une des revendications précédentes.

Fig.1a

Fig.1b

$T_{RR}$

$+Vt$

$-Vt$

$T_{REF}$

$T_{REF}$

Fig.2

E  H

100

111.1  110  111.2  109  108

$T_{REF1}$  $T_{REF2}$  $f_{RR\,(L)}$

$T_{REF}$

SIG  QRST  R  $f_{RR}$

Vt  CL

101  105  102  103  104  106  107

EP 0 793 979 B1

Fig.3

Fig.4

EP 0 793 979 B1